# EUROPEAN PATENT SPECIFICATION

(11) **EP 3 843 658 B1**
(45) Date of publication and mention of the grant of the patent: **17.04.2024**
(21) Application number: 18779112.4
(22) Date of filing: 31.08.2018
(51) Int. Cl.: A61F 2/07, A61F 2/95, A61M 25/01

(54) **SYSTEM FOR STEERING AN IMPLANTABLE MEDICAL DEVICE**
SYSTEM ZUR LENKUNG EINES IMPLANTIERBAREN MEDIZINPRODUKTS
SYSTÈME POUR DIRIGER UN DISPOSITIF MÉDICAL IMPLANTABLE

(43) Date of publication of application: 07.07.2021
(73) Proprietor: W. L. Gore & Associates, Inc., Newark, DE 19711 (US)
(72) Inventor: CATO, Milton C., Newark, DE 19711 (US); CHUNG, Karl R., Newark, DE 19711 (US)
(74) Representative: HGF
(86) International application number: PCT/US2018/049053
(87) International publication number: WO 2020/046364

(56) References cited:
- US-A1- 2009 216 308
- US-A1- 2009 259 291
- US-A1- 2010 094 401

## Description

### FIELD

The present invention relates to medical devices and methods for treating an anatomical space (e.g., vessels) of the body. More specifically, the invention relates to apparatuses, and systems that include an implantable medical device prosthesis that allows for accurate deployment in the anatomical space.

### BACKGROUND

Disease of the vasculature is increasingly common. Treatment of the vasculature may be difficult because of the tortuous nature and complexity of the vasculature. Aortic dissections, for example, commonly begin at or near the aortic valve root and continue to the ascending aorta and the aortic arch, and may also affect the upper part of the descending aorta. Medical devices implanted at a diseased state may be used for treatment of aortic dissections, aneurysms, and other diseases of the vasculature.
United States Patent Specification No. US 2009/259291A discloses a stent-graft with a tightenable loop element having a first end terminated in a slip knot or self-tightening knot and a second end which is received in and can slide in the knot. The knot is tied by a suture to the stent-graft so as to be fixed thereto. The loop is fitted to the stent-graft in a manner as to pass between the inside to the outside of the graft material and in such a manner that controlled curvature of the stent-graft is possible, in particular control of the overlapping of adjacent stents held within the zone of the loop. An introducer assembly is also disclosed which includes a control cannula able to the fixed to the stent-graft during the deployment procedure, as well as a mechanism of suture loops at the proximal end of the stent-graft for retaining this in a constricted form during the process of curving the latter during the deployment process.

It remains desirable to provide medical devices, systems and methods for repairing disease along the aorta (or other tortuous vessels) and also for repairing branches extending therefrom.

### SUMMARY

The invention is defined in the attached appended claims. The disclosure further describes the following examples.

According to one example ("Example 1"), a delivery system includes an implantable medical device; an actuation line configured to steer the implantable medical device during delivery thereof; a tether coupled to a portion of the implantable medical device arranged through the actuation line and configured to couple the actuation line to the implantable medical device; and a releasable knot including portions of the tether forming a first loop, a second loop, and a third loop with the third loop arranged through the second loop and the second loop arranged through the first loop and configured to couple the actuation line to the implantable medical device.

According to another example ("Example 2"), further to the delivery system of Example 1, the first loop is an eyelet formed by a first fold of the tether bonded at an overlapping portion of the first fold of the tether.

According to another example ("Example 3"), further to the delivery system of Example 2, the second loop is formed by a second fold of the tether and the third loop is formed by a third fold of the tether.

According to another example ("Example 4"), further to the delivery system of any one of Examples 1-3, the releasable knot is configured to release in response to tension applied to one end of the tether.

According to another example ("Example 5"), further to the delivery system of Example 1, the system also includes a fourth loop formed by a portion of the tether arranged through the third loop to form the releasable knot and the first loop is formed by a first fold of the tether, the second loop is formed by a second fold of the tether, the third loop is formed by a third fold of the tether, and the fourth loop is formed by a fourth fold of the tether.

According to another example ("Example 6"), further to the delivery system of Example 5, the releasable knot is configured to release in response to tension applied to both ends of the tether.

According to another example ("Example 7"), further to the delivery system of Example 5, a first end of the tether is arranged through the first loop, and a second end of the tether is arranged through the fourth loop, and the releasable knot is configured to release in response to tension applied to the second end of the tether.

According to another example ("Example 8"), further to the delivery system of any one of Examples 1-7, the releasable knot is configured to couple the actuation line to the implantable medical device without tension applied to the tether.

According to another example ("Example 9"), further to the delivery system of any one of Examples 1-8, the releasable knot is configured to resist separation between the actuation line and the implantable medical device in response to force applied to the actuation line.

According to one example ("Example 10"), a method of coupling an actuation line to an implantable medical device includes arranging the actuation line adjacent to the implantable medical device; routing a tether through an eyelet of the actuation line and coupling the tether to the implantable medical device; and forming a releasable knot that includes portions of the tether forming a first loop, a second loop, and a third loop with the third loop arranged through the second loop and the second loop arranged through the first loop and configured to couple the actuation line to the implantable medical device.

According to another example ("Example 11"), further to the method of Example 10, the method also includes visually inspecting the releasable knot to verify that the releasable knot couples the actuation line to the implantable medical device, and the releasable knot fails visual inspection by the releasable knot failing to maintain a knotted configuration and the releasable knot becoming at least partially unlooped.

According to another example ("Example 12"), further to the method of any one of Examples 10-11, forming the releasable knot includes forming the first loop by creating a first fold of the tether and bonding the tether at an overlapping portion of the first fold of the tether.

According to another example ("Example 13"), further to the method of any one of Examples 10-12, forming the releasable knot includes forming a fourth loop with a portion of the tether arranged through the third loop, wherein the first loop is formed by a first fold of the tether, the second loop is formed by a second fold of the tether, the third loop is formed by a third fold of the tether, and the fourth loop is formed by a fourth fold of the tether

According to another example ("Example 14"), further to the method of any one of Examples 10-11, the method also includes releasing the releasable knot by applying tension to one or both ends of the tether.

According to another example ("Example 15"), further to the method of any one of Examples 10-14, the releasable knot is configured to resist separation between the actuation line and the implantable medical device in response to force applied to the actuation line.

According to another example ("Example 16"), a delivery system includes an implantable medical device; a line having an eyelet; and a tether coupled to the implantable medical device and arranged through the eyelet of the line and configured to couple the line to the implantable medical device and including portions of the tether having a first loop, a second loop, and a third loop with the third loop arranged through the second loop and the second loop arranged through the first loop and configured to couple the line to the implantable medical device.

According to another example ("Example 17"), further to the delivery system of Example 16, the releasable knot is configured to resist separation between the line and the implantable medical device in response to force applied to the line.

According to another example ("Example 18"), further to the delivery system of Example 16, the releasable knot is configured to couple the line to the implantable medical device without tension applied to the tether.

According to another example ("Example 19"), further to the delivery system of Example 16, the tether is configured to release the loops by applying tension to one or both ends of the tether.

According to another example ("Example 20"), further to the delivery system of Example 16, the system also includes a fourth loop formed by a portion of the tether arranged through the third loop, wherein the first loop is formed by a first fold of the tether, the second loop is formed by a second fold of the tether, the third loop is formed by a third fold of the tether, and the fourth loop is formed by a fourth fold of the tether.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 shows an implantable medical device and an actuation line in accordance with an embodiment.
FIGS. 2A-2D show an example tether and steps in forming a releasable knot with portions of the tether in accordance with an embodiment.
FIGS. 3A-3E show another example tether and steps in forming a releasable knot with portions of the tether in accordance with an embodiment.
FIGS. 4A-4F show another example tether and steps in forming a releasable knot with portions of the tether in accordance with an embodiment.
FIGS. 5A-5E show side view illustrations of expandable device angulation relative to a target location in accordance with various aspects of the present disclosure.

### DETAILED DESCRIPTION

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward apparatuses, systems, and methods that include an implantable medical device that may be used in treatment of the vasculature. The implantable medical device is delivered to the vasculature using a delivery system. In addition, the implantable medical devices described herein may be substantially cylindrical, include a bifurcation, or have any of a variety of features. Further, the implantable medical devices may be configured to conform to the vasculature into which the implantable medical device is implanted, low-profile in order to facilitate delivery of the implantable medical device using a minimally invasive procedure (e.g., via transcatheter techniques), and able to withstand forces and other stresses that occur once implanted in the vasculature.

The delivery system may be configured to position and/or steer the implantable medical device for placement in the vasculature. To position and or steer the implantable medical device, the delivery system may include a line that changes position of the implantable medical device in response to a user applying force to the line. The line may be releasably coupled to the implantable medical device (e.g., to permit removal of the line to avoid trauma to the vasculature after the implantable medical device is delivered and positioned). As discussed in further detail below, a tether may be used to releasably couple the line to the implantable medical device. The tether may be formed into a releasable knot that is easily manufacturable and facilitates proper deployment. The releasable knots discussed herein avoid entangling with the line during release. In other instances, the tether may also be used to releasably couple other lines to the implantable medical device

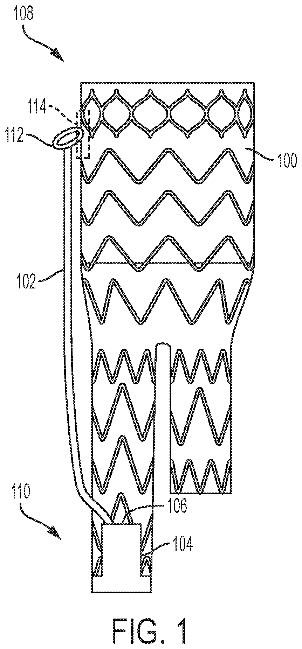

FIG. 1 shows an implantable medical device 100 and a line 102 in accordance with various aspects of the present disclosure. The implantable medical device 100 is releasably coupled to a delivery system for delivery of the implantable medical device 100 to a target location within a patient's vasculature. The delivery system may include a catheter 104 that includes a leading end 106 and a trailing end (not shown in FIG. 1). The implantable medical device 100 may be arranged near the leading end 106 of the catheter 104. The catheter 104 may extend through a lumen of the implantable medical device 100 toward and past a proximal end 108 of the implantable medical device 100. The catheter 104 may also include a tip (not shown) at the leading end 106. As shown in FIG. 1, the implantable medical device 100 includes stent and graft components, although the implantable medical device can take a variety of forms including a stent, graft, bifurcated implantable medical device, heart valve, and the like.

The implantable medical device 100 includes a proximal end 108, a distal end 110, and a flow lumen extending therebetween. The proximal end 108 of the implantable medical device 100 may be considered the end of the implantable medical device 100 that is closest to the target location within the patient's vasculature. The line 102 may be coupled to the implantable medical device 100 at one or more locations. As shown in FIG. 1, the line 102 is attached adjacent to or near the proximal end 108 of the implantable medical device 100 and accessible to a user of the delivery system.

As shown, the line 102 is coupled to the implantable medical device 100 via at least one tether 112. The tether 112 may be arranged through a portion of the implantable medical device 100 and through the line 102 to couple the line 102 to the implantable medical device 100. In certain instances, and as shown in FIG. 1, the at least one tether 112 is arranged through the implantable medical device 100 near or adjacent to the proximal end 108 of the implantable medical device 100. The at least one tether 112 may be a single tether, as shown in FIG. 1.

In some instances, the implantable medical device 100 may include a patch 114 attached or coupled to a surface (e.g., an exterior surface) of the implantable medical device 100. The patch 114 may include a layer of graft material that forms a lumen between the surface of the implantable medical device 100 and the graft material. The tether 112 may be arranged through the patch 114 to couple the line 102 to the implantable medical device 100.

In certain instances, the line 102 is an actuation line 102 configured to steer the implantable medical device 100 during delivery thereof. The actuation line 102 may include a column strength such that a user operating the delivery system may apply force to the actuation line 102 and bidirectionally steer (e.g., proximally and distally relative to the target location within the patient's vasculature) the implantable medical device 100. For example, the actuation line 102 may have a stiffness that is greater than a stiffness of the tether 112. The stiffness of the actuation line 102 and/or the location to which the actuation line 102 is coupled to the implantable medical device 100 may facilitate deploying and arranging the implantable medical device 100 relative to the target location within the patient's vasculature. For example, the implantable medical device 100 may be configured to deploy at a tortuous vessel having a curvature with at least one inflection point. In certain instances, the actuation line 102 is configured to maintain the proximal end 108 of the implantable medical device 100 approximately perpendicular to the inflection point in the curvature of the tortuous vessel during delivery of the implantable medical device 100.

The actuation line 102 may be uncoupled or released from the implantable medical device 100 subsequent to the implantable medical device 100 being positioned and deployed at the target location within the patient's vasculature and removed from the patient. In certain instances, the tether 112 is configured to remain coupled or threaded through the implantable medical device 100 after the actuation line 102 is released or uncoupled from the implantable medical device 100. The actuation line 102 can include an eyelet or opening in a distal end through which the tether 112 is arranged. In addition, the tether 112 may be formed from a bio-absorbable material that dissolves to release or uncouple the actuation line 102 from the implantable medical device 100. In other instances, the tether 112 is configured to be removed or unthreaded to uncouple the actuation line 102 from the implantable medical device 100.

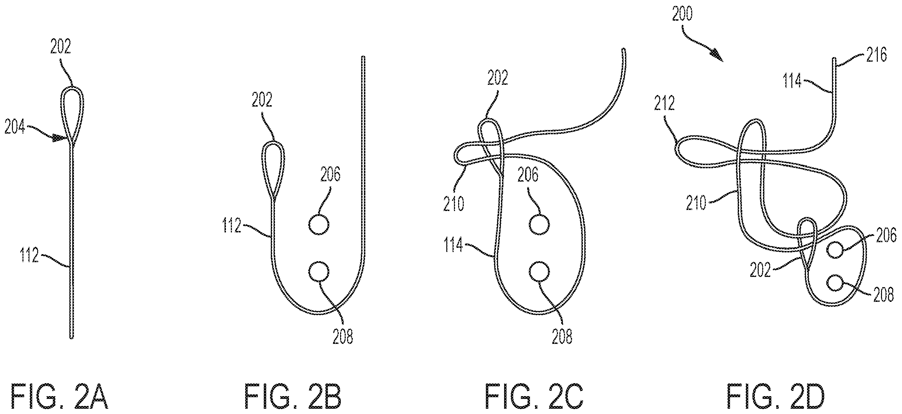

FIGS. 2A-D show an example tether 112 and steps in forming a releasable knot 200 with portions of the tether 112 in accordance with an embodiment. As explained in detail above with reference to FIG. 1, the tether 112 is coupled to a portion of an implantable medical device and arranged through a line (e.g., an actuation line) to couple the line to the implantable medical device. As shown in progressing through the steps shown in FIGS. 2A-D, the tether 112 forms a releasable knot, which is shown in a completed form in FIG. 2D. The tether 112 includes multiple loops in forming the releasable knot 200.

In certain instances, and as shown in FIG. 2A, the tether 112 includes first loop 202. The first loop 202 can be an eyelet that is formed by a first fold of the tether 112 that is bonded at an overlapping portion 204 of the first fold of the tether 112. The tether 112 may be bonded together by applying heat to bond the tether 112 to itself to form the first loop 202. The tether 112 is routed or arranged through a line and an implantable medical device to couple the line to the implantable medical device. For ease of illustration, element 206 is representative of one of the line and an implantable medical device and element 208 is representative of the other of the line and an implantable medical device. In certain instances, the element 206 may be the patch (shown in FIG. 1) or a hole in the implantable medical device and the element 208 may be an eyelet in the line.

As shown in FIG. 2B, the tether 112 is arranged about the element 206 and the element 208. In certain instances, the tether 112 is arranged through element 206 (the patch as shown in FIG. 1) and through an eyelet of the line. After being arranged through or about the element 206 and the element 208, the tether 112 may be folded to form a second loop 210 as shown in FIG. 2C. The second loop 210 of the tether 112 is then arranged through the first loop 202 of the tether 112.

In addition, and as shown in FIG. 2D, a third fold of the tether 112 may form a third loop 212. Subsequently, the third loop 212 is arranged through the second loop 210. Thus, the third loop 212 is arranged through the second loop 210, and the second loop 210 is arranged through the first loop 202 to form the releasable knot 200 as shown in FIG. 2D. In this configuration, the releasable knot 200 is configured to couple the actuation line to the implantable medical device.

After the multiple loops of the tether 112 are formed and arranged as discussed, tension may be applied to ends 214, 216 of the tether 112 to tighten the releasable knot 200. To release or untie the releasable knot 200, tension may be applied to one of the ends 214, 216.

Forming the releasable knot 200 in the manner discussed above may be manufacturable. In certain instances, the releasable knot 200 is formed or tied by hand. The steps to create the releasable knot 200 are relatively simple (e.g., not numerous, minimal crossover or loop formation in the tether 112). In addition, and in manufacturing or forming the releasable knot 200, it should be determined whether the releasable knot 200 is properly formed. The releasable knot 200 is properly formed when the releasable knot 200 couples the line to the implantable medical device. Forming the releasable knot 200 in the manner discussed above may also facilitate manufacturability by indicating whether the releasable knot 200 is properly formed. In this manner, the releasable knot 200 cannot be ineffectively formed. For example, if the steps in forming the releasable knot 200 are not followed, the releasable knot 200 will not maintain a knotted configuration (e.g., as shown in FIG. 2D) and the tether 112 will at least partially unloop. As a result, visually inspecting the releasable knot 200 to verify that the releasable knot 200 effectively couples the actuation line to the implantable medical device includes determining whether the releasable knot 200 maintains the knotted configuration or fails to do so.

In addition, the releasable knot 200 is configured to couple the actuation line to the implantable medical device without tension applied to the tether 112. After the releasable knot 200 is formed, tension does not need to be applied to hold the releasable knot 200 in the knotted configuration (e.g., as shown in FIG. 2D). An end 216 of the tether 112 may be coupled to a deployment handle (not shown) or a proximal portion of a catheter (e.g., as described above with reference to FIG. 1) such that a use may apply tension to the end 216 of the tether 112 or actuate a portion of the deployment handle that is coupled to the end 216 of the tether 112.

As noted above with reference to FIG. 1, the line (an actuation line) may steer the implantable medical device in response to a force applied to the actuation line. In certain instances, the force may be applied to the actuation line to bidirectionally steer (e.g., proximally and distally relative to the target location within the patient's vasculature) the implantable medical device. The releasable knot 200 is configured to maintain the knotted configuration to maintain the line coupled to the implantable medical device during steering. In certain instances, the releasable knot 200 is configured to resist separation between the (actuation) line and the implantable medical device in response to force applied to the actuation line. After the implantable medical device is aligned in a patient's vasculature (as noted above with reference to FIG. 1), the tether 112 is configured to release the second and third loops 210, 212 from the first loop 202 by applying tension to the end 216 of the tether 112.

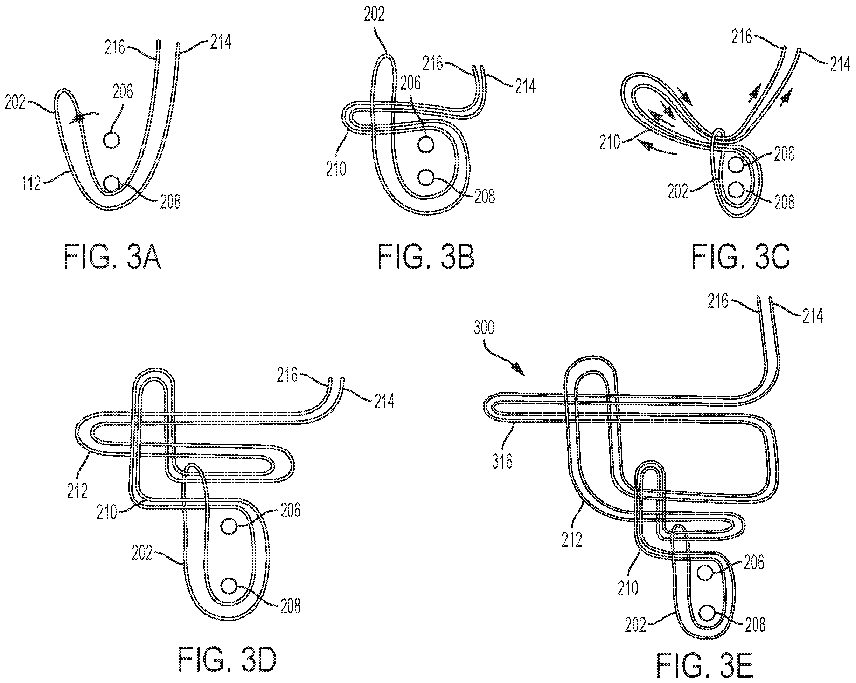

FIGS. 3A-E show another example tether 112 and steps in forming a releasable knot 300 with portions of the tether 112 in accordance with an embodiment. As explained in detail above with reference to FIG. 1, the tether 112 is coupled to a portion of an implantable medical device and arranged through a line (e.g., an actuation line) to couple the line to the implantable medical device. As shown in progressing through the steps shown in FIGS. 3A-E, the tether 112 forms a releasable knot 300, which is shown in a completed form in FIG. 3E. The tether 112 includes multiple loops in forming the releasable knot 300.

In certain instances, and as shown in FIG. 3A, the tether 112 includes first loop 202. The first loop 202 may be formed by a first fold of the tether 112. The tether 112 is routed or arranged through a line and an implantable medical device to couple the line to the implantable medical device. Similar to the illustration in FIGS. 2A-D, element 206 is representative of one of the line and an implantable medical device and element 208 is representative of the other of the line and an implantable medical device. In certain instances, the element 206 may be the patch (shown in FIG. 1) or a hole in the implantable medical device and the element 208 may be an eyelet in the line. The tether 112 is arranged about the element 206 and the element 208. In certain instances, the tether 112 is arranged through element 206 (the patch as shown in FIG. 1) and through an eyelet of the line. After being arranged through or about the element 206 and the element 208, the tether 112 may be folded to form a second loop 210 as shown in FIG. 3B. The second loop 210 of the tether 112 is then arranged through the first loop 202 of the tether 112.

FIG. 3C shows a pathway for the tether 112 in forming the second loop 210. As shown in FIG. 2C, both portions of folded over tether 112 are used to form the second loop 210. In addition, and as shown in FIGS. 3A-E, each of the loops formed by the tether 112 include both portions of a folded over tether 112. For example, and as shown in FIG. 3D, a third fold of the tether 112 may form a third loop 212. The third loop 212 is then arranged through the second loop 210. Subsequently, the third loop 212 is arranged through the second loop 210. As shown in FIG. 3E, a fourth fold of the tether 112 may form a fourth loop 316. The fourth loop 316 is then arranged through the third loop 212.

After the fourth loop 316 is arranged through the third loop 212, tension may be applied to ends 214, 216 of the tether 112 to tighten and complete the releasable knot 300. To release or untie the releasable knot 300, tension may be applied to both of the ends 214, 216. After the releasable knot 300 is formed, tension does not need to be applied to hold the releasable knot 300 in the knotted configuration (e.g., as shown in FIG. 3E). One or both of the ends 214, 216 may be coupled to a deployment handle (not shown) or a proximal portion of a catheter (e.g., as described above with reference to FIG. 1) such that a use may apply tension to one of the ends 214, 216 of the tether 112 or actuate a portion of the deployment handle that is coupled to one of the ends 214, 216 of the tether 112.

Forming the releasable knot 300 in the manner discussed above may be manufactureable. The steps to create the releasable knot 300 are relatively simple (e.g., not numerous, minimal crossover or loop formation in the tether 112). In addition and in manufacturing or forming the releasable knot 300, it should be determined whether the releasable knot 300 is properly formed. The releasable knot 300 is properly formed when the releasable knot 300 is able to couple the line to the implantable medical device. As a result, visually inspecting the releasable knot 200 to verify that the releasable knot 300 effectively couples the actuation line to the implantable medical device includes determining whether the releasable knot 200 maintains the knotted configuration or fails to do so.

As noted above with reference to FIG. 1, the line (an actuation line) may steer the implantable medical device in response to a force applied to the actuation line. In certain instances, the force may be applied to the actuation line to bidirectionally steer (e.g., proximally and distally relative to the target location within the patient's vasculature) the implantable medical device. The releasable knot 300 is configured to maintain the knotted configuration to maintain the line coupled to the implantable medical device during steering. In certain instances, the releasable knot 300 is configured to resist separation between the (actuation) line and the implantable medical device in response to force applied to the actuation line. After the implantable medical device is aligned in a patient's vasculature (as noted above with reference to FIG. 1), the tether 112 is configured to release the second and third loops 210, 212 from the first loop 202 by applying tension to the ends 214, 216 of the tether 112.

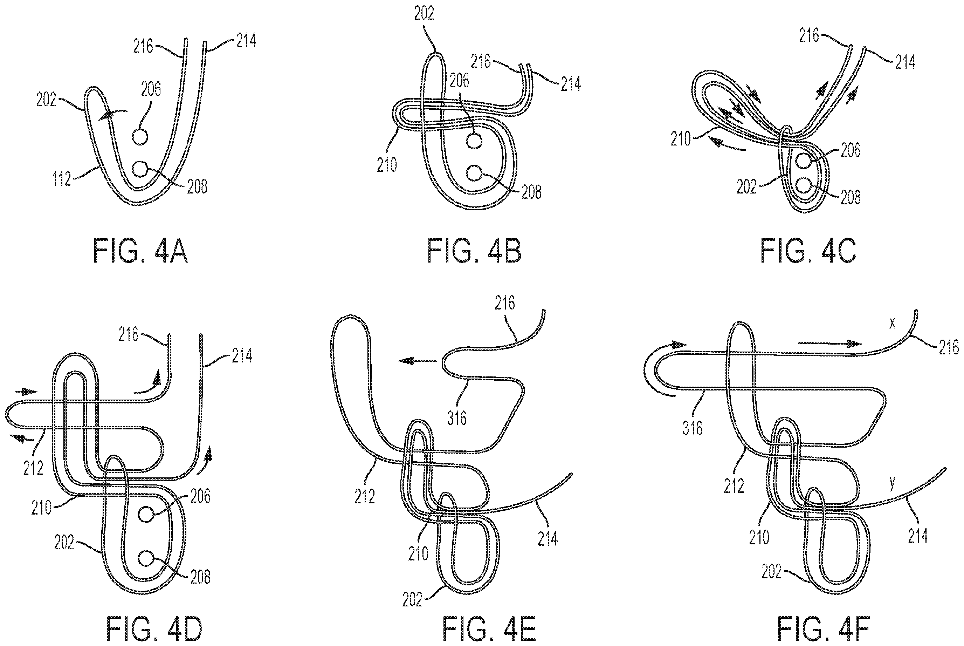

FIGS. 4A-F show another example tether and steps in forming a releasable knot with portions of the tether in accordance with an embodiment. As explained in detail above with reference to FIG. 1, the tether 112 is coupled to a portion of an implantable medical device and arranged through a line (e.g., an actuation line) to couple the line to the implantable medical device. As shown in progressing through the steps shown in FIGS. 4A-F, the tether 112 forms a releasable knot 400, which is shown in a completed form in FIG. 4F. As discussed in further detail below, the tether 112 forms a releasable knot 400 by way of multiple loops 202, 210, 212, 316.

In certain instances, and as shown in FIG. 4A, the tether 112 includes a first loop 202. The first loop 202 may be formed by a first fold of the tether 112. The tether 112 is routed or arranged through a line and an implantable medical device to couple the line to the implantable medical device. Similar to the illustration in FIGS. 2A-D and FIGS. 3A-E, element 206 is representative of one of the line and an implantable medical device and element 208 is representative of the other of the line and an implantable medical device. In certain instances, the element 206 may be the patch (shown in FIG. 1) or a hole in the implantable medical device and the element 208 may be an eyelet in the line. The tether 112 is arranged about the element 206 and the element 208. In certain instances, the tether 112 is arranged through element 206 (the patch as shown in FIG. 1) and through an eyelet of the line.

After being arranged through or about the element 206 and the element 208, the tether 112 may be folded to form a second loop 210 as shown in FIG. 4B. The second loop 210 of the tether 112 is then arranged through the first loop 202 of the tether 112. FIG. 4C shows a pathway for the tether 112 in forming the second loop 210. As shown in FIG. 4C, both portions of folded over tether 112 are used to form the second loop 210. Each of the first loop 202 and the second loop 210 formed by the tether 112 include both portions of a folded over tether 112. As shown in FIG. 4D, a third fold of the tether 112 that may form a third loop 212 includes one of the portions of the tether 112. As shown in FIG. 4D, end 214 of the tether 112 is arranged back through the first loop 202 while end 216 of the tether 112 is folded to form the third loop 212, which is arranged through the second loop 210.

Similar to the third loop 212 formed in the releasable knot 400, a fourth fold of the tether 112 may form a fourth loop 316 that includes the end 216 of the tether 112. The fourth loop 316 is then arranged through the third loop 212 as shown in FIGs. 4E-F. After the fourth loop 316 is arranged through the third loop 212, tension may be applied to ends 214, 216 of the tether 112 to tighten and complete the releasable knot 400.

To release or untie the releasable knot 400, tension may be applied to the end 216. In certain instances, the end 214 may be trimmed after the releasable knot 400 is formed. Further, the end 216 may be coupled to a deployment handle (not shown) or a proximal portion of a catheter (e.g., as described above with reference to FIG. 1) such that a user may apply tension to the end 216 of the tether 112 or actuate a portion of the deployment handle that is coupled to the end 216 of the tether 112. In addition, tension does not need to be applied to hold the releasable knot 400 in the knotted configuration (e.g., as shown in FIG. 4F).

Forming the releasable knot 400 in the manner discussed above may be manufactureable. The steps to create the releasable knot 400 are relatively simple (e.g., not numerous, minimal crossover or loop formation in the tether 112). In addition, and in manufacturing or forming the releasable knot 400, it should be determined whether the releasable knot 400 is properly formed. The releasable knot 400 is properly formed when the releasable knot 400 is able to couple the line to the implantable medical device. As a result, visually inspecting the releasable knot 200 to verify that the releasable knot 400 effectively couples the actuation line to the implantable medical device includes determining whether the releasable knot 200 maintains the knotted configuration or fails to do so.

As noted above with reference to FIG. 1, the line (an actuation line) may steer the implantable medical device in response to a force applied to the actuation line. The releasable knot 400 is configured to maintain the knotted configuration to maintain the line coupled to the implantable medical device during steering. In certain instances, the releasable knot 400 is configured to resist separation between the (actuation) line and the implantable medical device in response to force applied to the actuation line. After the implantable medical device is aligned in a patient's vasculature (as noted above with reference to FIG. 1), the tether 112 is configured to release the second and third loops 210, 212 from the first loop 202 by applying tension to one of the ends 214, 216 of the tether 112.

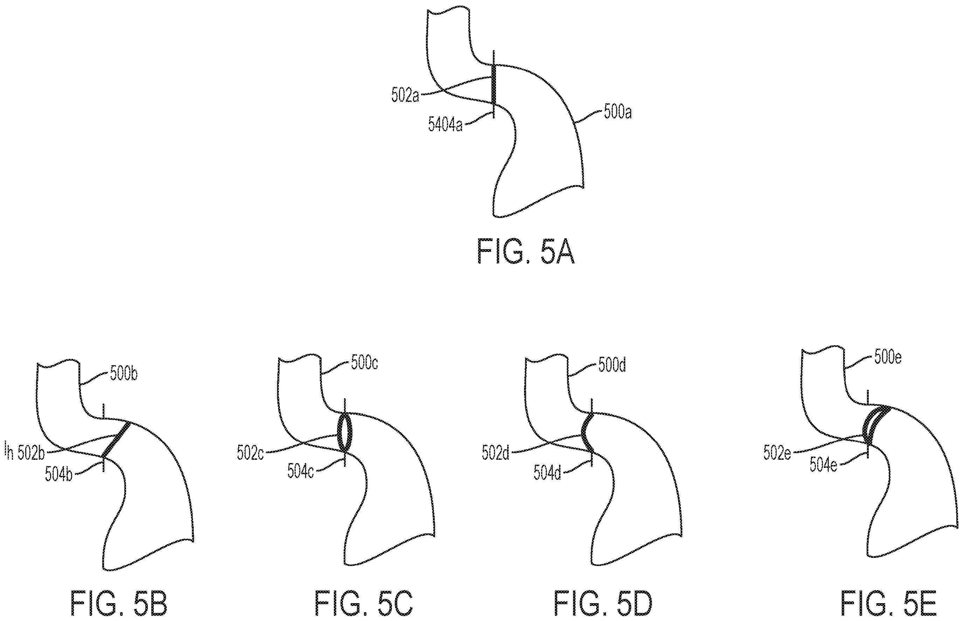

FIGS. 5A-E shows side view illustrations of expandable device angulation relative to a target location 500a-e in accordance with various aspects of the present disclosure. Each of FIGS. 5A-E show a side profile of a leading (or proximal) end 500a-e of an expandable device, consistent with various aspects of the present disclosure. In certain instances, the target location 500a-e may be at a tortuous vessel of a patient. The target location 500a-e into which the expandable device is implanted may have angulation (e.g., a curvature with at least one inflection point 504a-e). The target location 500a-e may be an angulated abdominal aortic aneurism (AAA), healthy tissue below the renal arteries, or other tortuous vessels. The releasable knots discussed herein enable actuation of the actuation wire at a location near a midpoint of the implantable device of distal to the midpoint (e.g., closer to the operator of the delivery system) on the implantable medical device.

In certain instances, one of the ends 502a-e of the expandable device may be deployed perpendicular to the inflection point (e.g., of a heathy vessel tissue) in the curvature of the tortuous vessel during delivery of the expandable device. Non-perpendicularity may negatively affect the ability of the expandable device to seal against the target location 500a-e. FIG. 5A shows the leading (or proximal) end 502a deployed perpendicular to the inflection point 504a. In certain instances, perpendicularity of the expandable device may be a function of device flatness, angulation, and rotational alignment. FIG. 5B shows the leading (or proximal) end 502b of an expandable device angled relative to the inflection point 504b of the target location 500b. FIG. 5C shows the leading (or proximal) end 502c of an expandable device rotated relative to the inflection point 504c of the target location 500c. FIG. 5D shows the leading (or proximal) end 502d of an expandable device deformed relative to the inflection point 504b of the target location 500d. FIG. 5E shows the leading (or proximal) end 502e of an expandable device deformed or flat, rotated, and angled relative to the inflection point 504e of the target location 500e.

Device deployment and performance can be enhanced by steering the device to an appropriate location while maintaining one of the ends of the expandable device perpendicular to the target location 500a-e (e.g., curvature of a vessel with at least one inflection point 504a-e) during and after deployment. The actuation lines and arrangements thereof discussed herein facilitate maintaining the expandable device perpendicular during and after deployment (as shown in FIG. 5A) and mitigate against non-perpendicular, angled, or flat deployment (as shown in FIGs. 5B-E).

The lines discussed herein may be formed from metallic, polymeric or natural materials such as stainless steels, cobalt-chromium alloys and nitinol. Further, the lines can also be formed from high strength polymer fibers such as ultra high molecular weight polyethylene fibers (e.g., SpectraTM., Dyneema PurityTM., etc.) or aramid fibers (e.g., TechnoraTM, etc.).

The graft components of the implantable medical device and/or the patch may be made up of any material which is suitable for use as a graft in the chosen body lumen and being resistant to expansion as discussed herein. The graft components may be composed of the same or different materials. Furthermore, the graft components may include multiple layers of material that can be the same material or different material. In one embodiment, said materials can be used in combination and assembled together to comprise a graft. The graft materials used in a stent graft can be extruded, coated or formed from wrapped films, or a combination thereof. Polymers, biodegradable and natural materials can be used for specific applications.

Examples of synthetic polymers include, but are not limited to, nylon, polyacrylamide, polycarbonate, polyformaldehyde, polymethylmethacrylate, polytetrafluoroethylene, polytrifluorochlorethylene, polyvinylchloride, polyurethane, elastomeric organosilicon polymers, polyethylene, polypropylene, polyurethane, polyglycolic acid, polyesters, polyamides, their mixtures, blends and copolymers are suitable as a graft material. In one embodiment, said graft is made from a class of polyesters such as polyethylene terephthalate including DACRON^{®}, and MYLAR ^{®} and polyaramids such as KEVLAR ^{®}., polyfluorocarbons such as polytetrafluoroethylene (PTFE) with and without copolymerized hexafluoropropylene (TEFLON^{®}, or GORE-TEX^{®}.), and porous or nonporous polyurethanes. In another embodiment, said graft comprises expanded fluorocarbon polymers (especially PTFE) materials described in British Pat. Nos. 1,355,373; 1,506,432; or 1,506,432 or in U.S. Pat. Nos. 3,953,566; 4,187,390; or 5,276,276. Included in the class of preferred fluoropolymers are polytetrafluoroethylene (PTFE), fluorinated ethylene propylene (FEP), copolymers of tetrafluoroethylene (TFE) and perfluoro(propyl vinyl ether) (PFA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinyfluoride (PVF). Especially preferred, because of its widespread use in vascular prostheses, is ePTFE. In another embodiment, said graft comprises a combination of said materials listed above. In another embodiment, said graft is substantially impermeable to bodily fluids. Said substantially impermeable graft can be made from materials that are substantially impermeable to bodily fluids or can be constructed from permeable materials treated or manufactured to be substantially impermeable to bodily fluids (e.g. by layering different types of materials described above or known in the art). In another embodiment, said outermost tube comprises ePTFE. In another embodiment, said innermost tube comprises ePTFE. In another embodiment, said innermost and outermost tube comprises ePTFE film that has been wrapped into a tube. In another embodiment, said secondary stent is covered with any of the material disclosed herein or known in the art. In another embodiment, the secondary stent covering comprises ePTFE.

Additional examples of graft materials include, but are not limited to, vinylidinefluoride/hexafluoropropylene hexafluoropropylene (HFP), tetrafluoroethylene (TFE), vinylidenefluoride, 1-hydropentafluoropropylene, perfluoro(methyl vinyl ether), chlorotrifluoroethylene (CTFE), pentafluoropropene, trifluoroethylene, hexafluoroacetone, hexafluoroisobutylene, fluorinated poly(ethylene-co-propylene (FPEP), poly(hexafluoropropene) (PHFP), poly(chlorotrifluoroethylene) (PCTFE), poly(vinylidene fluoride (PVDF), poly(vinylidene fluoride-co-tetrafluoroethylene) (PVDF-TFE), poly(vinylidene fluoride-co-hexafluoropropene) (PVDF-HFP), poly(tetrafluoroethylene-co-hexafluoropropene) (PTFE-HFP), poly(tetrafluoroethylene-co-vinyl alcohol) (PTFE-VAL), poly(tetrafluoroethylene-co-vinyl acetate) (PTFE-VAC), poly(tetrafluoroethylene-co-propene) (PTFEP) poly(hexafluoropropene-co-vinyl alcohol) (PHFP-VAL), poly(ethylene-co-tetrafluoroethylene) (PETFE), poly(ethylene-co-hexafluoropropene) (PEHFP), poly(vinylidene fluoride-co-chlorotrifluoroe-thylene) (PVDF-CTFE), and combinations thereof, and additional polymers and copolymers described in U.S. Publication 2004/0063805. Additional polyfluorocopolymers include tetrafluoroethylene (TFE)/perfluoroalkylvinylether (PAVE). PAVE can be perfluoromethylvinylether (PMVE), perfluoroethylvinylether (PEVE), or perfluoropropylvinylether (PPVE), as described in U.S. Publication 2006/0198866 and U.S. Pat. No. 7,049,380. Other polymers and copolymers include, polylactide, polycaprolacton-glycolide, polyorthoesters, polyanhydrides; poly-aminoacids; polysaccharides; polyphosphazenes; poly(ether-ester) copolymers, e.g., PEO-PLLA, or blends thereof, polydimethyl-siolxane; poly(ethylene-vingylacetate); acrylate based polymers or copolymers, e.g., poly(hydroxyethyl methylmethacrylate, polyvinyl pyrrolidinone; fluorinated polymers such as polytetrafluoroethylene; cellulose esters and any polymer and copolymers described in U.S. Publication 2004/0063805.

The graft components, as discussed herein, may be attached to the self-expanding stent elements by using a coupling member that is generally a flat ribbon or tape having at least one generally flat surface. In certain instances, the tape member is made from expanded PTFE (ePTFE) coated with an adhesive. The adhesive may be a thermoplastic adhesive. In certain instances, the thermoplastic adhesive may be fluorinated ethylene propylene (FEP). More specifically, an FEP-coated side of the ePTFE may face toward and contact an exterior surface of the self-expanding stent and graft component, thus attaching the self-expanding stent to the graft component. Materials and method of attaching a stent to the graft is discussed in U.S. Pat. No. 6,042,602 to Martin.

The stent component(s) discussed herein can be fabricated from a variety of biocompatible materials. These materials may include 316L stainless steel, cobalt-chromium-nickel-molybdenum-iron alloy ("cobalt-chromium"), other cobalt alloys such as L605, tantalum, Nitinol, or other biocompatible metals. In certain instances, as discussed in detail above, the stent (and graft) may be self-expanding. In other instances, the prosthesis may be balloon expandable.

The stent component(s) discussed herein may be constructed from a reasonably high strength material, i.e., one which is resistant to plastic deformation when stressed. In one embodiment, the stent component(s) comprise a line which is helically wound around a mandrel having pins arranged thereon so that the helical turns and undulations can be formed simultaneously. Other constructions may also be used. In certain instances, the stent component(s) are made from a super-elastic alloy. There are a variety of disclosures in which super-elastic alloys such as nitinol are used in stents. See for example, U.S. Pat. Nos. 4,503,569, to Dotter; 4,512,338, to Balko et al.; 4,990,155, to Wilkoff; 5,037,427, to Harada, et al.; 5,147,370, to MacNamara et al.; 5,211,658, to Clouse; and 5,221,261, to Termin et al.

A variety of materials variously metallic, super elastic alloys, such as Nitinol, are suitable for use in the stent component(s). Primary requirements of the materials are that they be suitably springy even when fashioned into very thin sheets or small diameter lines. Various stainless steels which have been physically, chemically, and otherwise treated to produce high springiness are suitable as are other metal alloys such as cobalt chrome alloys (e.g., ELGILOY^{®}), platinum/tungsten alloys, and especially the nickel-titanium alloys generically known as "nitinol".

## Claims

1. A delivery system comprising:
an implantable medical device (100);
an actuation line (102) configured to steer the implantable medical device during delivery thereof;
a tether (112) coupled to a portion of the implantable medical device arranged through the actuation line and configured to couple the actuation line to the implantable medical device; and
a releasable knot (200) including portions of the tether (112) forming a first loop (202), a second loop (210), and a third loop (212) with the third loop (212) arranged through the second loop (210) and the second loop (210) arranged through the first loop (202) and configured to couple the actuation line (102) to the implantable medical device (100).

2. The delivery system of claim 1, wherein the first loop (202) is an eyelet formed by a first fold of the tether (112) bonded at an overlapping portion of the first fold of the tether; and optionally, wherein the second loop (212) is formed by a second fold of the tether and the third loop is formed by a third fold of the tether.

3. The delivery system of any one of claims 1-2, wherein the releasable knot (200) is configured to release in response to tension applied to one end of the tether (112).

4. The delivery system of claim 1, further comprising a fourth loop formed by a portion of the tether arranged through the third loop to form the releasable knot, wherein the first loop is formed by a first fold of the tether, the second loop is formed by a second fold of the tether, the third loop is formed by a third fold of the tether, and the fourth loop is formed by a fourth fold of the tether; and optionally,
wherein the releasable knot is configured to release in response to tension applied to both ends of the tether; or
wherein a first end of the tether is arranged through the first loop, and a second end of the tether is arranged through the fourth loop, and the releasable knot is configured to release in response to tension applied to the second end of the tether.

5. The delivery system of any one of claims 1-4, wherein the releasable knot is configured to couple the actuation line to the implantable medical device without tension applied to the tether; or
wherein the releasable knot is configured to resist separation between the actuation line and the implantable medical device in response to force applied to the actuation line.

6. A method of coupling an actuation line to an implantable medical device, the method including:
arranging the actuation line adjacent to the implantable medical device;
routing a tether through an eyelet of the actuation line and coupling the tether to the implantable medical device; and
forming a releasable knot that includes portions of the tether forming a first loop, a second loop, and a third loop with the third loop arranged through the second loop and the second loop arranged through the first loop and configured to couple the actuation line to the implantable medical device.

7. The method of claim 6, further including visually inspecting the releasable knot to verify that the releasable knot couples the actuation line to the implantable medical device, and the releasable knot fails visual inspection by the releasable knot failing to maintain a knotted configuration and the releasable knot becoming at least partially unlooped.

8. The method of any one of claims 6-7, wherein forming the releasable knot includes forming the first loop by creating a first fold of the tether and bonding the tether at an overlapping portion of the first fold of the tether.

9. The method of any one of claims 6-8, wherein forming the releasable knot (200) includes forming a fourth loop (316) with a portion of the tether (112) arranged through the third loop (212), wherein the first loop (202) is formed by a first fold of the tether, the second loop (210) is formed by a second fold of the tether, the third loop (212) is formed by a third fold of the tether, and the fourth loop (316) is formed by a fourth fold of the tether.

10. The method of any one of claims 6-9, further comprising releasing the releasable knot (300) by applying tension to one or both ends (214,216) of the tether (112).

11. The method of any one of claims 6-10, wherein the releasable knot (200, 300) is configured to resist separation between the actuation line (102) and the implantable medical device (100) in response to force applied to the actuation line (102).

12. The delivery system of claim 1 comprising:
the actuation line (102) having an eyelet; and
the tether (112) coupled to the implantable medical device (100) and arranged through the eyelet of the line (102) and configured to couple the actuation line (102) to the implantable medical device (100) and including portions of the tether (112) having a first loop (202), a second loop (210), and a third loop (212) with the third loop (212) arranged through the second loop (210) and the second loop (210) arranged through the first loop (202) and configured to couple the line (102) to the implantable medical device (100).

13. The delivery system of claim 12, wherein the releasable knot (200) is configured to resist separation between the actuation line (102) and the implantable medical device (102) in response to force applied to the line; or
wherein the releasable knot (200) is configured to couple the actuation line (102) to the implantable medical device (100) without tension applied to the tether (112).

14. The delivery system of claim 12, wherein the tether (112) is configured to release the loops by applying tension to one or both ends of the tether (112).

15. The delivery system of claim 12, further comprising a fourth loop (316) formed by a portion of the tether (112) arranged through the third loop (212), wherein the first loop (202) is formed by a first fold of the tether, the second loop (210) is formed by a second fold of the tether, the third loop (212) is formed by a third fold of the tether, and the fourth loop (316) is formed by a fourth fold of the tether.

## Patentansprüche

1. Zuführungssystem, umfassend:
eine implantierbare medizinische Vorrichtung (100);
ein Betätigungsseil (102), das dazu konfiguriert ist, die implantierbare medizinische Vorrichtung während ihrer Zuführung zu lenken;
ein Halteband (112), das mit einem Abschnitt der implantierbaren medizinischen Vorrichtung gekoppelt ist, durch das Betätigungsseil angeordnet ist und dazu konfiguriert ist, das Betätigungsseil mit der implantierbaren medizinischen Vorrichtung zu koppeln; und
einen lösbaren Knoten (200), der Abschnitte des Haltebandes (112) enthält, die eine erste Schlaufe (202), eine zweite Schlaufe (210) und eine dritte Schlaufe (212) bilden, wobei die dritte Schlaufe (212) durch die zweite Schlaufe (210) angeordnet ist und die zweite Schlaufe (210) durch die erste Schlaufe (202) angeordnet ist, und dazu konfiguriert ist, das Betätigungsseil (102) mit der implantierbaren medizinischen Vorrichtung (100) zu koppeln.

2. Zuführungssystem nach Anspruch 1, wobei die erste Schlaufe (202) eine Öse ist, die durch eine erste Schlinge des Haltebandes (112) gebildet wird, die an einen überlappenden Abschnitt der ersten Schlinge des Haltebandes gebunden ist; und optional, wobei die zweite Schlaufe (212) durch eine zweite Schlinge des Haltebandes gebildet wird und die dritte Schlaufe durch eine dritte Schlinge des Haltebandes gebildet wird.

3. Zuführungssystem nach einem der Ansprüche 1-2, wobei der lösbare Knoten (200) dazu konfiguriert ist, sich als Reaktion darauf, dass auf ein Ende des Haltebandes (112) ein Zug ausgeübt wird, zu lösen.

4. Zuführungssystem nach Anspruch 1, ferner eine vierte Schlaufe umfassend, die durch einen Abschnitt des Haltebandes gebildet wird, der durch die dritte Schlaufe angeordnet ist, um den lösbaren Knoten zu bilden, wobei die erste Schlaufe durch eine erste Schlinge des Haltebandes gebildet wird, die zweite Schlaufe durch eine zweite Schlinge des Haltebandes gebildet wird, die dritte Schlaufe durch eine dritte Schlinge des Haltebandes gebildet wird und die vierte Schlaufe durch eine vierte Schlinge des Haltebandes gebildet wird; und optional
wobei der lösbare Knoten dazu konfiguriert ist, sich als Reaktion darauf, dass ein Zug auf beide Enden des Haltebandes ausgeübt wird, zu lösen; oder
wobei ein erstes Ende des Haltebandes durch die erste Schlaufe angeordnet ist und ein zweites Ende des Haltebandes durch die vierte Schlaufe angeordnet ist und der lösbare Knoten dazu konfiguriert ist, sich als Reaktion darauf, dass ein Zug auf das zweite Ende des Haltebandes ausgeübt wird, zu lösen.

5. Zuführungssystem nach einem der Ansprüche 1-4, wobei der lösbare Knoten dazu konfiguriert ist, das Betätigungsseil mit der implantierbaren medizinischen Vorrichtung zu koppeln, ohne dass Zug auf das Halteband ausgeübt wird; oder
wobei der lösbare Knoten dazu konfiguriert ist, einer Trennung zwischen dem Betätigungsseil und der implantierbaren medizinischen Vorrichtung als Reaktion darauf, dass eine Kraft auf das Betätigungsseil ausgeübt wird, zu widerstehen.

6. Verfahren zum Koppeln eines Betätigungsseils mit einer implantierbaren medizinischen Vorrichtung, wobei das Verfahren Folgendes enthält:
Anordnen des Betätigungsseils neben der implantierbaren medizinischen Vorrichtung;
Führen eines Haltebandes durch eine Öse des Betätigungsseils und Koppeln des Haltebandes mit der implantierbaren medizinischen Vorrichtung; und
Bilden eines lösbaren Knotens, der Abschnitte des Haltebandes enthält, die eine erste Schlaufe, eine zweite Schlaufe und eine dritte Schlaufe bilden, wobei die dritte Schlaufe durch die zweite Schlaufe angeordnet ist und die zweite Schlaufe durch die erste Schlaufe angeordnet ist, und dazu konfiguriert ist, das Betätigungsseil mit der implantierbaren medizinischen Vorrichtung zu koppeln.

7. Verfahren nach Anspruch 6, ferner visuelles Überprüfen des lösbaren Knotens enthaltend, um sicherzustellen, dass der lösbare Knoten das Betätigungsseil mit der implantierbaren medizinischen Vorrichtung koppelt, und wobei der lösbare Knoten die visuelle Überprüfung nicht besteht, falls der lösbare Knoten scheitert, eine geknotete Konfiguration aufrechtzuerhalten, und sich die Schlaufen des lösbaren Knotens mindestens teilweise auflösen.

8. Verfahren nach einem der Ansprüche 6-7, wobei das Bilden des lösbaren Knotens Bilden der ersten Schlaufe durch Erzeugen einer ersten Schlinge des Haltebandes und Binden des Haltebandes an einen überlappenden Abschnitt der ersten Schlinge des Haltebandes enthält.

9. Verfahren nach einem der Ansprüche 6-8, wobei das Bilden des lösbaren Knotens (200) Bilden einer vierten Schlaufe (316) enthält, wobei ein Abschnitt des Haltebandes (112) durch die dritte Schlaufe (212) angeordnet wird, wobei die erste Schlaufe (202) durch eine erste Schlinge des Haltebandes gebildet wird, die zweite Schlaufe (210) durch eine zweite Schlinge des Haltebandes gebildet wird, die dritte Schlaufe (212) durch eine dritte Schlinge des Haltebandes gebildet wird und die vierte Schlaufe (316) durch eine vierte Schlinge des Haltebandes gebildet wird.

10. Verfahren nach einem der Ansprüche 6-9, ferner Lösen des lösbaren Knotens (300) durch Ausüben eines Zugs auf ein oder beide Enden (214,216) des Haltebandes (112) umfassend.

11. Verfahren nach einem der Ansprüche 6-10, wobei der lösbare Knoten (200, 300) dazu konfiguriert ist, einer Trennung zwischen dem Betätigungsseil (102) und der implantierbaren medizinischen Vorrichtung (100) als Reaktion darauf, dass eine Kraft auf das Betätigungsseil (102) ausgeübte wird, zu widerstehen.

12. Zuführungssystem nach Anspruch 1, umfassend:
das Betätigungsseil (102), das eine Öse aufweist; und
das Halteband (112), das mit der implantierbaren medizinischen Vorrichtung (100) gekoppelt ist und durch die Öse des Seils (102) angeordnet ist und dazu konfiguriert ist, das Betätigungsseil (102) mit der implantierbaren medizinischen Vorrichtung (100) zu koppeln, und Abschnitte des Haltebandes (112) enthält, die eine erste Schlaufe (202), eine zweite Schlaufe (210) und eine dritte Schlaufe (212) bilden, wobei die dritte Schlaufe (212) durch die zweite Schlaufe (210) angeordnet ist und die zweite Schlaufe (210) durch die erste Schlaufe (202) angeordnet ist, und dazu konfiguriert ist, das Seil (102) mit der implantierbaren medizinischen Vorrichtung (100) zu koppeln.

13. Zuführungssystem nach Anspruch 12, wobei der lösbare Knoten (200) dazu konfiguriert ist, einer Trennung zwischen dem Betätigungsseil (102) und der implantierbaren medizinischen Vorrichtung (102) als Reaktion darauf, dass eine Kraft auf das Seil ausgeübt wird, zu widerstehen; oder
wobei der lösbare Knoten (200) dazu konfiguriert ist, das Betätigungsseil (102) mit der implantierbaren medizinischen Vorrichtung (100) zu koppeln, ohne dass Spannung auf das Halteband (112) ausgeübt wird.

14. Zuführungssystem nach Anspruch 12, wobei das Halteband (112) dazu konfiguriert ist, die Schlaufen durch Ausüben eines Zugs auf ein oder beide Enden des Haltebandes (112) zu lösen.

15. Zuführungssystem nach Anspruch 12, ferner eine vierte Schlaufe (316) umfassend, die durch einen Abschnitt des Haltebandes (112) gebildet wird, der durch die dritte Schlaufe (212) angeordnet wird, wobei die erste Schlaufe (202) durch eine erste Schlinge des Haltebandes gebildet wird, die zweite Schlaufe (210) durch eine zweite Schlinge des Haltebandes gebildet wird, die dritte Schlaufe (212) durch eine dritte Schlinge des Haltebandes gebildet wird und die vierte Schlaufe (316) durch eine vierte Schlinge des Haltebandes gebildet wird.

## Revendications

1. Système d'administration comprenant :
un dispositif médical implantable (100) ;
une ligne d'actionnement (102) configurée pour diriger le dispositif médical implantable pendant son administration ;
une attache (112) couplée à une partie du dispositif médical implantable agencée à travers la ligne d'actionnement et configurée pour coupler la ligne d'actionnement au dispositif médical implantable ; et
un noeud libérable (200) comprenant des parties de l'attache (112) formant une première boucle (202), une deuxième boucle (210) et une troisième boucle (212), la troisième boucle (212) étant agencée à travers la deuxième boucle (210) et la deuxième boucle (210) étant agencée à travers la première boucle (202) et configurée pour coupler la ligne d'actionnement (102) au dispositif médical implantable (100).

2. Système d'administration selon la revendication 1, dans lequel la première boucle (202) est un oeillet formé par un premier pli de l'attache (112) liée au niveau d'une partie chevauchante du premier pli de l'attache ; et facultativement, dans lequel la deuxième boucle (212) est formée par un deuxième pli de l'attache et la troisième boucle est formée par un troisième pli de l'attache.

3. Système d'administration selon l'une quelconque des revendications 1 à 2, dans lequel le noeud libérable (200) est configuré pour se libérer en réponse à une tension appliquée à une extrémité de l'attache (112).

4. Système d'administration selon la revendication 1, comprenant en outre une quatrième boucle formée par une partie de l'attache agencée à travers la troisième boucle pour former le noeud libérable, dans lequel la première boucle est formée par un premier pli de l'attache, la deuxième boucle est formée par un deuxième pli de l'attache, la troisième boucle est formée par un troisième pli de l'attache, et la quatrième boucle est formée par un quatrième pli de l'attache ; et facultativement,
dans lequel le noeud libérable est configuré pour se libérer en réponse à une tension appliquée aux deux extrémités de l'attache ; ou
dans lequel une première extrémité de l'attache est agencée à travers la première boucle, et une seconde extrémité de l'attache est agencée à travers la quatrième boucle, et le noeud libérable est configuré pour se libérer en réponse à une tension appliquée à la seconde extrémité de l'attache.

5. Système de distribution selon l'une quelconque des revendications 1 à 4, dans lequel le noeud libérable est configuré pour coupler la ligne d'actionnement au dispositif médical implantable sans qu'une tension ne soit appliquée à l'attache ; ou
dans lequel le noeud libérable est configuré pour résister à la séparation entre la ligne d'actionnement et le dispositif médical implantable en réponse à une force appliquée à la ligne d'actionnement.

6. Procédé de couplage d'une ligne d'actionnement à un dispositif médical implantable, le procédé comprenant :
l'agencement de la ligne d'actionnement adjacente au dispositif médical implantable ;
l'acheminement d'une attache à travers un oeillet de la ligne d'actionnement et le couplage de l'attache au dispositif médical implantable ; et
la formation d'un noeud libérable qui comprend des parties de l'attache formant une première boucle, une deuxième boucle et une troisième boucle, la troisième boucle étant agencée à travers la deuxième boucle et la deuxième boucle étant agencée à travers la première boucle et configurée pour coupler la ligne d'actionnement au dispositif médical implantable.

7. Procédé selon la revendication 6, comprenant en outre l'inspection visuelle du noeud libérable pour vérifier que le noeud libérable couple la ligne d'actionnement au dispositif médical implantable, et le noeud libérable échoue l'inspection visuelle lorsque le noeud libérable ne parvient pas à maintenir une configuration nouée et que le noeud libérable devient au moins partiellement défait.

8. Procédé selon l'une quelconque des revendications 6 et 7, dans lequel la formation du noeud libérable comprend la formation de la première boucle en créant un premier pli de l'attache et en liant l'attache au niveau d'une partie de chevauchement du premier pli de l'attache.

9. Procédé selon l'une quelconque des revendications 6 à 8, dans lequel la formation du noeud libérable (200) comprend la formation d'une quatrième boucle (316) avec une partie de l'attache (112) agencée à travers la troisième boucle (212), dans lequel la première boucle (202) est formée par un premier pli de l'attache, la deuxième boucle (210) est formée par un deuxième pli de l'attache, la troisième boucle (212) est formée par un troisième pli de l'attache, et la quatrième boucle (316) est formée par un quatrième pli de l'attache.

10. Procédé selon l'une quelconque des revendications 6 à 9, comprenant en outre la libération du noeud libérable (300) en appliquant une tension à l'une ou aux deux extrémités (214, 216) de l'attache (112).

11. Procédé selon l'une quelconque des revendications 6 à 10, dans lequel le noeud libérable (200, 300) est configuré pour résister à la séparation entre la ligne d'actionnement (102) et le dispositif médical implantable (100) en réponse à une force appliquée à la ligne d'actionnement (102).

12. Système d'administration selon la revendication 1, comprenant :
la ligne d'actionnement (102) comportant un oeillet ; et
l'attache (112) couplée au dispositif médical implantable (100) et agencée à travers l'œillet de la ligne (102) et configurée pour coupler la ligne d'actionnement (102) au dispositif médical implantable (100) et comprenant des parties de l'attache (112) comportant une première boucle (202), une deuxième boucle (210) et une troisième boucle (212), la troisième boucle (212) étant agencée à travers la deuxième boucle (210) et la deuxième boucle (210) étant agencée à travers la première boucle (202) et configurée pour coupler la ligne (102) au dispositif médical implantable (100).

13. Système d'administration selon la revendication 12, dans lequel le noeud libérable (200) est configuré pour résister à la séparation entre la ligne d'actionnement (102) et le dispositif médical implantable (102) en réponse à une force appliquée à la ligne ; ou
dans lequel le noeud libérable (200) est configuré pour coupler la ligne d'actionnement (102) au dispositif médical implantable (100) sans qu'une tension ne soit appliquée à l'attache (112).

14. Système d'administration selon la revendication 12, dans lequel l'attache (112) est configurée pour libérer les boucles en appliquant une tension à une ou aux deux extrémités de l'attache (112).

15. Système d'administration selon la revendication 12, comprenant en outre une quatrième boucle (316) formée par une partie de l'attache (112) agencée à travers la troisième boucle (212), dans lequel la première boucle (202) est formée par un premier pli de l'attache, la deuxième boucle (210) est formée par un deuxième pli de l'attache, la troisième boucle (212) est formée par un troisième pli de l'attache, et la quatrième boucle (316) est formée par un quatrième pli de l'attache.
